(19) Europäisches Patentamt
European Patent Office
Office européen des brevets

(11) **EP 1 845 947 B1**

(12) **EUROPEAN PATENT SPECIFICATION**

(45) Date of publication and mention
of the grant of the patent:
**31.12.2008 Bulletin 2009/01**

(51) Int Cl.:
***A61K 9/16*** *(2006.01)*   ***A61K 9/20*** *(2006.01)*

(21) Application number: **06701525.5**

(22) Date of filing: **17.01.2006**

(86) International application number:
**PCT/IB2006/000189**

(87) International publication number:
**WO 2006/079922 (03.08.2006 Gazette 2006/31)**

(54) **FAST-DISINTEGRATING MICROPOROUS BINDER AND PROCESS FOR MAKING IT**

SCHNELL ZERFALLENDES MIKROPORÖSES BINDEMITTEL UND HERSTELLUNGSVERFAHREN DAFÜR

LIANTS MICROPOREUX A DESINTEGRATION RAPIDE ET PROCÉDÉ POUR LE FABRIQUER

(84) Designated Contracting States:
**AT BE BG CH CY CZ DE DK EE ES FI FR GB GR
HU IE IS IT LI LT LU LV MC NL PL PT RO SE SI
SK TR**

(30) Priority: **28.01.2005 US 648231 P**

(43) Date of publication of application:
**24.10.2007 Bulletin 2007/43**

(73) Proprietor: **Pfizer Products Inc.**
**Groton, CT 06340-5146 (US)**

(72) Inventors:
 • **CREW, Marshall David**
 **Bend, OR 97701 (US)**
 • **FALK, Richard Frank**
 **Bend Research Inc.**
 **Bend, OR 97701 (US)**
 • **FRIESEN, Dwayne Thomas**
 **Bend Research Inc.**
 **Bend, OR 97701 (US)**
 • **KONAGURTHU, Sanjay**
 **Bend Research Inc.**
 **Bend, OR 97701 (US)**
 • **RAY, Roderick Jack**
 **Bend Research Inc.**
 **Bend, OR 97701 (US)**

(74) Representative: **Westwood, Joanna**
 **Wilson Gun**
 **Charles House**
 **148/9 Great Charles Street**
 **Birmingham B3 3HT (GB)**

(56) References cited:
 **EP-A- 1 369 109**       **WO-A-20/04100857**

**Description**

BACKGROUND OF THE INVENTION

Binders are routinely used in the pharmaceutical arts to impart cohesive qualities to powdered material. Remington: The Science and Practice of Pharmacy, 20th Ed. (2000). Commonly used binders include cellulosic polymers such as methyl cellulose, carboxymethyl cellulose and hydroxypropyl methyl cellulose; microcrystalline cellulose; starch; sugars such as sucrose, glucose, dextrose, lactose; and gums such as guar gum and tragacanth gum. When conventional binders are used to make compressed solid dosage forms (e.g., tablets, caplets, pills), the resulting dosage forms are strong and have high strength (or low friability). These properties make conventional binders ideal for compressive force applications.

However, because of their high strength, tablets made using conventional binders tend to take several minutes or longer to disintegrate when introduced to an aqueous environment, such as the GI tract. When fast dissolution or fast disintegration of the tablet is desired, large amounts of disintegrants or other materials must be included in such tablets. However, such disintegrant-type materials often detract from the desired cohesiveness of the powdered material being compressed into a tablet. As a result, a substantial amount of binder must be used. The net result is a large tablet that does not dissolve or disintegrate as rapidly as desired.

A number of attempts have been made to solve the foregoing problems. EP 1 008 353 discloses a spray-dried pharmaceutical binder powder of calcium hydrogen phosphate and a sugar or sugar alcohol that is stated to be rapidly disintegrable in water or in the mouth. However, there is no disclosure concerning the binder's porosity or quantitative water solubility.

WO 96/17579 discloses a coprecipitated pharmaceutical binder of an acid such as citric acid and an N-vinyl lactam non-cellulosic polymer such as polyvinylpyrrolidone that is ground into a fine powder and that is non-friable and readily water-soluble.

WO 98/52541 discloses granules used in tableting drugs where the granules are made by spray-drying or precompacting alkaline earth metal salts and carbohydrates. To form the granules by spray-drying, a slurry of calcium carbonate, an aqueous-soluble polysaccharide such as starch or maltodextrin, and a sugar alcohol is spray-dried.

U.S. Patent No. 6,177,104 discloses a particulate support matrix for pharmaceutical formulations consisting of two aqueous-soluble polypeptides such as hydrolyzed and non-hydrolyzed gelatin and a "bulking agent" that may be a sugar alcohol that are spray-dried together.

WO 97/47304 discloses a "fast-dissolving" tablet of galanthamine and a carrier comprising a spray-dried mixture of microcrystalline cellulose and lactose monohydrate, together with a disintegrant. However, although the microcrystalline cellulose component is hydrophilic, it is not aqueous-soluble. This may account for the fact that the tablets are not truly fast dissolving, but have a dissolution rate in water of 80% dissolved after 30 minutes.

WO 01/10418 A1 discloses a tablet matrix comprising a spray-dried mixture of a protein such as fish gelatin and a sugar that is then dry-blended with a "binding polymer" of polyethylene glycol (PEG) having a molecular weight of 1000 to 1,000,000. To form the fast-dissolving tablet, a drug is blended with the matrix, and the blend is compressed into a tablet. The tablet is sintered at 50° to 100° C to melt and thereby disperse the PEG, then allowed to cool to resolidify the PEG. The principal drawback of this process is the sintering step, which limits the application to drugs having melting points much higher than the temperatures generated during sintering.

WO 2004/100 857 A and EP 1369109 A do not disclose the specific polymers of claim 1.

BRIEF SUMMARY OF THE INVENTION

The inventors have overcome the foregoing limitations of prior art binders by designing a microporous binder that can be used to make fast-disintegrating or fast-dissolving dosage forms of virtually any drug. The microporous nature of the binder allows for rapid ingress of water into the dosage form, followed by rapid disintegration or dissolution of the dosage form. The binder acts as a channel for the mass transfer of water and as an aqueous-soluble or partially aqueous-soluble dosage form component.

In a first aspect, the invention provides a fast-disintegrating dosage form comprising a drug and a microporous binder. The microporous binder is free from the drug and comprises a plurality of particles, each of the particles comprising an aqueous-soluble cellulosic polymer and a wicking agent. The aqueous-soluble cellulosic polymer and wicking agent together comprise at least about 60 wt% of the microporous binder. The microporous binder has a porosity of at least about 70%.

In a second aspect, the invention provides a process of making the microporous binder of the invention.

Chief among the advantages provided by the invention is the provision of a fast-disintegrating binder that facilitates rapid disintegration and/or dissolution of compressed dosage forms such as tablets. Without wishing to be bound by theory, it is believed that the wicking agent acts to rapidly absorb or wick water into the binder particles. The combination of the wicking agent and the microporous nature of the binder particles result in rapid water absorption, allowing the aqueous-soluble cellulosic polymer in the particles to rapidly disintegrate and/or dissolve.

BRIEF DESCRIPTION OF THE DRAWING

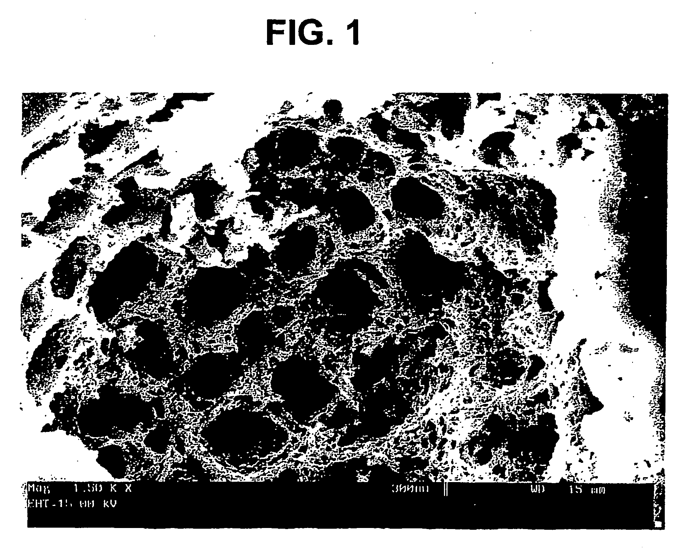

FIG. 1 is a 1500x scanning electron microphotograph of the microporous binder of the present invention showing a high degree of porosity.

DETAILED DESCRIPTION OF PREFERRED EMBODIMENTS

The fast-disintegrating dosage form of the invention comprises a drug and a microporous binder. As used herein, the term "binder" is used conventionally, meaning a material that is used to impart cohesive qualities to a powdered material. See for example, Remington: The Science and Practice of Pharmacy, 20th Edition (2000). Prior to incorporation into a dosage form, the microporous binder is free from the drug. As used herein and in the claims, the term "free from the drug" means that the microporous binder, prior to incorporation into a dosage form, contains less than about 10 wt% drug. Preferably, the microporous binder, prior to incorporation into a dosage form, contains less than about 5 wt% drug, and more preferably less than about 1 wt% drug. The amount of drug in the microporous binder prior to incorporation into a dosage form can be determined using standard analytical methods, such as by high-performance liquid chromatography (HPLC). In one embodiment, the microporous binder is substantially free from the drug, meaning that the amount of drug in the microporous binder prior to incorporation into a dosage agent is below the detection limits of standard analytical techniques.
Described below are the microporous binder, its characteristics, exemplary processes for making the binder, suitable drugs for use in dosage forms containing the binder, and exemplary dosage forms made using the binder.

**Microporous Binders**

The binders of the invention comprise microporous particles of an aqueous-soluble cellulosic polymer and a wicking agent resulting from a four-component composition that has undergone liquid-liquid or solid-liquid phase separation, as described in greater detail below. By "phase separation" is meant that a single-phase composition is placed under conditions where it is no longer stable as a single-phase solution, and therefore undergoes phase separation into at least two phases: (a) a polymer- and first solvent-rich phase, and (b) a wicking agent- and second solvent-rich phase. By "liquid-liquid" phase separation is meant that the two phases formed are both liquid. By "solid-liquid" phase separation is meant that at least one of the phases formed is liquid and at least one of the phases formed is solid. By "microporous" is meant the particles contain pores with a diameter of about 10 $\mu$m or less. Preferably, the pores have a diameter of about 5 $\mu$m or less, and more preferably about 1 $\mu$m or less. A particle is considered to be microporous if it contains pores in this size range, even if it contains larger pores, sometimes referred to as macropores. Thus, a particle is considered to be microporous if it contains pores with a diameter of about 10 $\mu$m or less. In addition, it is preferred that the micropores are distributed substantially uniformly throughout the material. As used herein, "aqueous-soluble" in reference to the polymer in the inventive binder means not only that the polymer is soluble in water in the conventional sense of going into solution, but also that the polymer may be water-dispersible in the sense of disintegrating or breaking up into small particles in an aqueous environment. By "small particles" is meant particles with an average diameter sufficiently small that they do not feel "gritty" in the mouth. Thus, the particles may have an average diameter of less than about 50 $\mu$m, or even less than about 10 $\mu$m. Preferably, the polymer has an aqueous solubility of at least about 0.1 mg/mL over at least a portion of the pH range of 1 to 8. For example, a polymer that has a solubility of less than 0.1 mg/mL at a pH of less than 4, but a solubility of 0.1 mg/mL or more at a pH of 5 or greater is suitable for use with the invention. Preferably the polymer has an aqueous solubility of at least about 0.2 mg/mL, more preferably at least about 0.3 mg/mL, and most preferably at least about 0.4 mg/mL over at least a portion of the pH range of 1 to 8.
The aqueous-soluble polymers for use in forming the microporous binders of the invention are aqueous-soluble ionizable cellulosic polymers selected from hydroxypropylmethyl cellulose phthalate (HPMCP), hydroxypropylmethyl cellulose acetate succinate (HPMCAS), carboxymethylethyl cellulose (CMEC), cellulose acetate phthalate (CAP), and cellulose acetate trimellitate (CAT). By "cellulosic" is meant a cellulose polymer that has been modified by reaction of at least a portion of the hydroxyl groups on the saccharide repeating units with a compound to form an ester or an ether substituent.
Mixtures of such polymers may also be used. A particularly preferred polymer is HPMCAS.
The wicking agent used in the microporous binders of the invention acts to facilitate the rapid ingress of water into the binder when placed in an aqueous use environment. The wicking agent should be soluble in water over at least a portion of the pH range of 1 to 8. Preferably, the wicking agent has an aqueous solubility of at least about 1 mg/mL over at least a portion of the pH range of 1 to 8. Preferably, the wicking agent has an aqueous solubility of at least about 2 mg/mL, more preferably at least about 5 mg/mL, and most preferably at least about 10 mg/mL over at least a portion of the pH range of 1 to 8. Examples of wicking agents suitable for use in the microporous binders of the present invention include polyols, such as sugars and sugar alcohols; salts; organics; low-molecular-weight polymers (i.e., less than about 2000 daltons); and mixtures thereof. Specific examples of wicking agents include sugars, such as sucrose, glucose, dextrose, lactose, and trehalose; sugar alcohols, such as mannitol, sorbitol, and xylitol; salts, such as calcium chloride, sodium citrate, sodium bicarbonate, and sodium carbonate; organics, such as citric acid; low-molecular weight polymers, such as polyethylene glycol, polyvinyl pyrrolidone, and poloxamers; and mixtures thereof. A particularly preferred wicking agent is sucrose.

The microporous particles contain both an aqueous-soluble cellulosic polymer and a wicking agent. The aqueous-soluble cellulosic polymer and wicking agent together comprise the major portion of the microporous particles, meaning that the polymer and wicking agent make up at least about 60 wt% of the total mass of the particles. The polymer and wicking agent may make up an even greater percentage of the particles, such as 70 wt%, 80 wt%, 90 wt%, 95 wt%, or 98 wt% or more of the total mass of the particles. In one embodiment, the microporous particles consist essentially of an aqueous-soluble cellulosic polymer and a wicking agent.

The mass ratio of polymer to wicking agent is preferably chosen so that, when the microporous particles are formed by a liquid-liquid or solid-liquid phase separation process, a continuous polymer phase is formed. Hence polymer: wicking agent mass ratios may vary from 30:70 to as high as 90:10. In terms of percentage polymer present versus the total amount of polymer and wicking agent in the microporous particles, there should be at least about 30 wt% polymer, preferably at least about 50 wt% polymer, and most preferably at least about 60 to 80 wt% polymer. As used in the specification and claims, the term "about" when used in reference to a numerical value means the value $\pm$ 10% of the value.

The degree of porosity of the inventive microporous particles is at least about 70%, more preferably at least about 75%, and most preferably at least about 80%. The porosity of the microporous particles may be measured by standard quantitative techniques for determining the porosity of materials, such as by mercury porosimetry, well known in the art. The microporous particles also have a tapped specific volume of at least about 4 cc/g. This is equivalent to a tapped density of no more than about 0.25 g/cc. Porosity of the microporous particles of the invention may be determined by mercury porosimetry using, for example, an AutoPore IV Mercury Porosimeter, commercially available from Micrometrics, Inc., of Norcross, Georgia. A sample of the microporous particles is added to the porosimeter and is immersed in mercury and pressure is applied to cause mercury to penetrate the pores of the particles. The change in mercury volume or intrusion volume as a function of pressure is measured and then converted to log differential intrusion volume and plotted against pore size. From this, the relative contribution of void volume as a function of pore size may be quantified as a percentage of porosity.

## Forming the Microporous Binders

The microporous binders of the invention are preferably formed by a process (described *infra*) in which a single-phase feed solution undergoes liquid-liquid or solid-liquid phase separation prior to formation of the solid particles. In the process, a single phase feed solution is formed by dissolving the polymer and the wicking agent in two solvents, referred to herein as a first solvent and a second solvent. This four-component single phase liquid feed solution is then subjected to conditions that result in liquid-liquid or solid-liquid phase separation, forming two phases, namely, (1) a polymer- and first solvent-rich phase and (2) a wicking agent-and second solvent-rich phase. Preferably, the polymer-and first solvent-rich phase is a continuous phase, while the wicking agent- and second solvent-rich phase is a non-continuous phase. Alternatively, both phases may be continuous, forming a so-called bi-continuous two-phase mixture. Following phase separation, the two phases are solidified. Generally, the solvents are then removed, resulting in the formation of microporous particles comprising the polymer and the wicking agent. However, in some cases one or both solvents may remain, at least partially in the particles. This is particularly true when one or both of the solvents have a melting point above room temperature; that is, greater than about 25°C. In such cases, the solvent may solidify within the microporous binder.

Without wishing to be bound by any theory or mechanism of formation, it is believed that because the polymer-and first solvent-rich phase is continuous, when the solvent is at least partially removed from the solution to form solid particles, the particles consist of microporous polymer, wherein the micropores may contain at least a portion of the wicking agent. In this fashion the wicking agent functions to (*i*) increase the amount of aqueous-soluble material in the binder and (*ii*) increase the rate of water imbibition into the binder, which promotes faster disintegration and/or dissolution.

Thus, in one aspect, the invention provides a process for preparing a microporous binder comprising the steps: (a) forming a single phase liquid feed solution comprising an aqueous-soluble polymer, a wicking agent, a first solvent, and a second solvent; (b) causing the feed solution to undergo liquid-liquid or solid-liquid phase separation to form two-phases; and (c) removing a substantial portion of the first solvent and the second solvent from the two phases to form

4

the solid microporous binder. In one embodiment, steps (b) and (c) may be conducted in a single continuous process, such as by spray-drying. As to step (c), by removing a "substantial portion" of the first solvent and second solvent from the two phases is meant that sufficient amounts of the first solvent and second solvent are removed from the two phases so as to form a solid material. As mentioned above, if one or both of the solvents solidify at ambient temperatures, much of the solvent may remain in the material and still meet the requirement that the material solidifies. Solidification of the two-phases may occur when the first solvent, the second solvent, or both solvents are removed from the material. Generally, a substantial portion of at least one of the first or second solvents is removed so that the amount of the solvent present in the solid microporous binder is less than about 10 wt%.

Preferred methods of conducting step (b) (causing such liquid-liquid or solid-liquid phase separation) include (*i*) decreasing the ratio of the first solvent and the second solvent; (ii) reducing the total amount of solvent (but not the ratio of first solvent to second solvent); (*iii*) changing the temperature of the feed solution; and (*iv*) any combination of (*i*), (*ii*), and (*iii*). These methods are described in detail below.

The process may result in the formation of a plurality of microporous binder particles. For example, the liquid feed solution may be atomized during the process, resulting in the formation of a plurality of particles of the microporous binder. Examples of such processes, described below, include spray-drying and spray-cooling. Preferably, the particles have a $D_{90}$ of less than about 500 $\mu$m, preferably less than about 300 $\mu$m, and most preferably less than about 150 $\mu$m. As used herein, "$D_{90}$" is the diameter corresponding to the diameter of particles that make up 90% of the total volume of particles of equal or smaller diameter. In other words, if $D_{90}$ is equal to 500 $\mu$m, 90 vol% of the particles have a diameter less than or equal to 500 $\mu$m. However, if the particles are too small, the microporous binder may be difficult to handle in downstream processing. Thus, it is also preferred that the particles have a $D_{10}$ of about 1 $\mu$m or more, preferably about 5 $\mu$m or more, and most preferably of about 10 $\mu$m or more, where $D_{10}$ is the diameter corresponding to the diameter of particles that make up 10% of the total volume of particles of equal or smaller volume.

When the process used to form the microporous binder results in the formation of microporous binder that has larger than desirable particle size, the microporous binder may be further processed to form a plurality of microporous binder particles. For example, the process may be conducted in an extruder so as to form a continuous rod or large particles (greater than about 1 $\mu$m) of microporous binder. Alternatively, the single-phase liquid feed may be cast in the form of a sheet or film. Such rods, large particles, or sheets may be subsequently processed into a powder. Exemplary processes for reducing particle size include grinding, milling, and screening processes well known in the art. See Remington: The Science and Practice of Pharmacy, 20th Edition (2000). Conversely, if there are more small particles than desired, the microporous binder can be granulated using dry or wet granulation processes well known in the art.

*Causing Phase Separation*

*Method (i)*

In the case of Method (*i*) for causing phase separation (decreasing the ratio of the first and second solvents), the first solvent is selected so that it has a greater volatility than the second solvent. The four-component feed solution containing the aqueous-soluble cellulosic polymer, the wicking agent, the first solvent and the second solvent is placed under conditions where the first solvent evaporates more rapidly than the second solvent due to their difference in volatility, thus reducing the ratio of the first solvent to the second solvent. Ultimately, the ratio of the first solvent to the second solvent is sufficiently low that at the temperature of the mixture, the mixture is no longer stable as a single phase solution, and the single-phase feed solution therefore undergoes liquid-liquid or solid-liquid phase separation, forming two phases: (a) a polymer- and first solvent-rich phase and (b) a wicking agent- and second solvent-rich phase. The two phases formed may both be liquid, or one of the phases may be liquid and the other solid. Preferably, the two phases are both liquid. Following phase separation, a sufficient amount of the first solvent and/or second solvent are removed, resulting in solidification of the microporous binder comprising the polymer and wicking agent.

Suitable solvents for the first solvent preferably have a boiling point of 150°C or less, have a relatively low toxicity and are pharmaceutically acceptable. Exemplary first solvents include alcohols, such as methanol, ethanol, the various isomers of propanol and the various isomers of butanol; ketones, such as acetone, methyl ethyl ketone; esters, such as methyl acetate, ethyl acetate, and propyl acetate; ethers, such as tetrahydrofuran, methyl tetrahydrofuran, 1,3-dioxane, and 1,4-dioxane; nitriles, such as acetonitrile; water; and mixtures thereof. Mixtures of solvents, such as 50% methanol and 50% acetone, can also be used. Preferred first solvents include acetone, methyl ethyl ketone, methanol, ethanol, methyl acetate, ethyl acetate, tetrahydrofuran, 1,3-dioxolane, and mixtures thereof. Most preferred first solvents include acetone, methanol, ethanol, ethyl acetate, and mixtures thereof.

The second solvent should be less volatile than the first solvent, meaning that its boiling point is higher than the first solvent boiling point. Preferably, the boiling point of the second solvent is at least about 10°C higher than the boiling point of the first solvent. Examples of second solvents include those listed above for the first solvent. The preferred solvent for use as the second solvent is water.

Examples of Method (*i*) for causing a liquid-liquid or solid-liquid phase separation by altering the ratio of the first solvent to the second solvent include removal of the two solvents from the particles by an evaporative process selected from spray- drying, simple evaporation, rotoevaporation, multiple effect evaporation and wiped film evaporation, all well known in the pharmaceutical arts. In each of these cases, the four-component feed solution containing the aqueous-soluble cellulosic polymer, the wicking agent, the first solvent, and the second solvent is placed under conditions where the more volatile first solvent is removed from the feed solution faster than is the less-volatile second solvent. Phase separation then occurs, and as the remaining first solvent and second solvent are removed, a microporous material is formed. Such processes may be performed in a single continuous process, or they may be performed in a batch or non-continuous process.

In a preferred method, the microporous binder is formed by spray-drying. In this process, a single phase liquid feed solution is atomized into a chamber to form liquid droplets. The higher-volatility first solvent evaporates more rapidly from the droplet than the lower-volatility second solvent. As a result, the ratio of the first solvent to the second solvent changes, and the liquid droplet undergoes phase separation. The conditions in the spray-drying apparatus are selected such that the first solvent and second solvent continue to evaporate from the phase separated droplet, ultimately forming a solid particle of the microporous binder.

The term "spray-drying" is used conventionally and broadly refers to processes involving breaking up liquid mixtures into small droplets (atomization) and rapidly removing solvent from the mixture in a spray-drying apparatus where there is a strong driving force for evaporation of solvent from the droplets. Spray-drying processes and spray-drying equipment are described generally in Perry's Chemical Engineers' Handbook, pages 20-54 to 20-57 (Sixth Ed. 1984). More details on spray-drying processes and equipment are reviewed by Marshall, "Atomization and Spray-Drying," 50 Chem. Eng. Prog. Monogr. Series 2 (1954), and Masters, Spray Drying Handbook (Fourth Ed.1985). The strong driving force for solvent evaporation is generally provided by maintaining the partial pressure of solvent in the spray-drying apparatus well below the vapor pressure of the solvent at the temperature of the drying droplets. This is accomplished by (1) maintaining the pressure in the spray-drying apparatus at a partial vacuum (*e.g.*, 0.01 to 0.50 atm); or (2) mixing the liquid droplets with a warm drying gas; or (3) both (1) and (2). In addition, at least a portion of the heat required for evaporation of solvent may be provided by heating the spray solution.

Various types of nozzles can be used to atomize the spray solution, thereby introducing the spray solution into the spray-dry chamber as a collection of small droplets. Essentially any type of nozzle may be used to spray the solution as long as the droplets that are formed are sufficiently small that (1) a sufficient amount of the first solvent is removed from the droplet for phase separation to occur, and (2) following phase separation, a sufficient amount of the remaining first solvent and second solvent are removed that the so-formed particles are sufficiently dry that they do not stick to or coat the spray-drying chamber wall.

Although the maximum droplet size varies widely as a function of the size, shape and flow pattern within the spray-dryer, generally droplets should be less than about 500 $\mu$m in diameter when they exit the nozzle. Examples of types of nozzles that may be used to form the solid compositions include the two-fluid nozzle, the fountain-type nozzle, the flat fan-type nozzle, the pressure nozzle and the rotary atomizer. Preferably, the atomizer produces droplets with an average droplet diameter of 50 $\mu$m or larger, with less than about 10 vol% of the droplets having a size less than about 10 $\mu$m. Such large drops are preferred to ensure phase separation occurs prior to solidification of the droplet. In a preferred embodiment, a pressure nozzle is used, as disclosed in detail in commonly assigned copending U.S. Application No. 10/351,568, the disclosure of which is incorporated herein by reference.

It is also preferred that the droplets formed have a uniform drop size distribution. The "Span," sometimes referred to in the art as the Relative Span Factor or RSF, is a dimensionless parameter indicative of the uniformity of the drop size distribution, and is defined as

$$Span = \frac{D_{90} - D_{10}}{D_{50}} ,$$

where $D_{90}$ is the diameter corresponding to the diameter of droplets that make up 90% of the total liquid volume containing droplets of equal or smaller diameter; $D_{10}$ is the diameter corresponding to the diameter of droplets that make up 10% of the total liquid volume containing droplets of equal or smaller diameter, and $D_{50}$ is the diameter corresponding to the diameter of drops that make up 50% of the total liquid volume containing drops of equal of smaller diameter. For example, if $D_{90}$ is equal to 100 $\mu$m, 90 vol% of the droplets have a diameter less than or equal to 100 $\mu$m. Generally, the lower the Span, the more narrow the droplet size distribution produced by the atomizing means, which in turn generally leads to a narrower particle size distribution for the dried particles, resulting in improved flow characteristics. Preferably, the Span of the droplets produced by the atomizing means of the present invention is less than about 3, more preferably less than about 2, and most preferably less than about 1.5. Methods to determine droplet size and droplet size distribution are well known in the art and can be found in Lefebvre, Atomization and Sprays (1989).

The spray solution can be delivered to the atomizer at a wide range of temperatures and flow rates. Generally, the spray solution temperature can range anywhere from just above the freezing point of the spray solution to about 20°C above its ambient pressure boiling point (by pressurizing the solution) and in some cases even higher. Spray solution flow rates to the atomizer can vary over a wide range depending on the viscosity of the spray solution, type of nozzle, spray-dryer size and spray-dry conditions such as the inlet temperature and flow rate of the drying gas. Generally, the energy for evaporation of solvent from the spray solution in a spray-drying process comes primarily from the drying gas.

The drying gas can, in principle, be essentially any gas, but for safety reasons and to minimize undesirable oxidation of the materials in the particles, an inert gas such as nitrogen, nitrogen-enriched air or argon is typically utilized. The drying gas is typically introduced into the drying chamber at a temperature between about 60° and about 300°C and preferably between about 80° and about 240°C.

The large surface-to-volume ratio of the droplets and the large driving force for evaporation of solvent leads to rapid solidification times for the droplets following phase separation. Solidification times should be less than about 20 seconds, preferably less than about 10 seconds, and more preferably less than 1 second. In a preferred embodiment, the height and volume of the spray-dryer are adjusted to provide sufficient time for the droplets to dry prior to impinging on an internal surface of the spray-dryer, as described in detail in U.S. Patent No. 6,763,607, the disclosure of which is incorporated herein by reference.

Following solidification, the solid microporous particles typically stay in the spray-drying chamber for about 5 to 60 seconds, further evaporating solvent therefrom. The final first solvent and second solvent content of the microporous particles as they exit the dryer should be low. Generally, the total solvent content of the particles as they leave the spray-drying chamber should be less than about 10 wt% and preferably less than about 2 wt% based on the total mass of the particles.

In another process of Method (*i*), the microporous binder is formed by an extrusion process. By "extrusion process" is meant a process where the feed solution is processed in an extruder, such as a twin-screw extruder well known in the art. In one embodiment, the feed solution is first formed and then fed to the extruder. In another embodiment, the feed solution is prepared in the extruder by feeding the water-soluble cellulosic polymer, the wicking agent, the first solvent and the second solvent to the extruder. The extrusion process is designed such that the ratio of the first-solvent to the second solvent is altered either in the extruder by partial venting of the solvents, or as the extruded material exits the extruder. The conditions in the extruder are selected such that the extruder produces the microporous binder, typically in the form of large particles or as rods. The large particles or rods can subsequently be processed to produce a powder with the desired particle size.

Alternatively, the feed solution may be cast as a film to form a sheet of material using procedures well known in the art. The feed solution may be cast onto an appropriate substrate or onto a surface. As the film or sheet is formed, solvent is removed, altering the ratio of the first solvent to the second solvent, resulting the formation of the microporous binder. The microporous binder made using this method is typically in the form of a flat sheet or film, which can subsequently be milled to obtain a microporous binder with the desired particle size.

*Method (ii)*

In the case of Method (*ii*) for causing phase separation (reducing total solvent content), the feed solution is processed so that the total amount of solvent in the material is reduced, without changing the ratio of the first solvent to the second solvent. As the amount of solvent in the feed solution decreases, the mixture is no longer stable as a single phase solution, and therefore the feed solution undergoes phase separation, forming two phases. Following phase separation, a sufficient amount of the first and second solvents are removed, resulting in solidification of the microporous binder.

The same solvents described above for Method (*i*) may be used for Method (*ii*), except that the second solvent need not be less volatile than the first solvent; indeed, the first and second solvents may have the same volatility.

To form the microporous binders of the invention following phase separation of the feed solution by Method (*ii*), the same processes described above in connection with Method (*i*) may be used.

*Method (iii)*

In the case of Method (*iii*) for causing phase separation of the four-component feed solution (changing the temperature of the feed solution), the first solvent and the second solvent are selected such that the four-component feed solution is initially a single phase. Upon heating or cooling of the solution, phase separation occurs. In one embodiment, the four-component feed solution is a single phase at elevated temperatures (i.e., temperatures greater than room temperature). In another embodiment, the four-component feed solution is a single phase at room temperature. In either case, when the temperature of the single phase feed solution is changed, either by allowing it to cool to ambient temperature or by refrigeration, or by heating, the feed solution undergoes phase separation

Once phase separation has occurred, the first solvent and the second solvent are removed to form a solid microporous material. The solvents can be removed using evaporative techniques, such as those described above for Method (*i*), including spray-drying, evaporation, rotoevaporation, multiple-effect evaporation, and wiped film evaporation.

Alternatively, the solvents can be removed by a lyophilization process, also known as freeze-drying, well known in the art. In this process, following phase separation, the feed solution is cooled below its freezing point, forming a solid mass containing the polymer, wicking agent, the first solvent, and the second solvent. The first solvent and the second solvent are then removed from the frozen solid by sublimation with a vacuum using processes well known in the art. See, for example, Remington: The Science and Practice of Pharmacy, 20th Edition (2000).

In all of these phase separation methods utilizing a temperature change, in general the same parameters, same solvents and same mass ratios of polymer to wicking agent may be utilized as mentioned above in connection with achieving phase separation by Method (*i*). However, with phase separation by temperature changes, there is no need for a difference in volatility of the first solvent and the second solvent.

*Method (iv)*

As to achieving phase separation by Method (*iv*), which is a combination of Methods (*i*), (*ii*) and (*iii*), in general this method includes both evaporation of the two solvents and one or more heating or cooling steps. Exemplary processes include a spray-cooling process where the feed solution is atomized into a chilled chamber to cause phase separation as the solvent ratio is altered and the solution is cooled.

**Drugs**

In one aspect, the invention provides a pharmaceutical composition comprising a drug, the binder described herein, and one or more optional excipients. The term "drug" is conventional, denoting a compound having beneficial prophylactic and/or therapeutic properties when administered to an animal, especially to humans.

The drug may be in a crystalline, semi-crystalline, semi-ordered, or amorphous state or a combination of these states or states that lie between. The term "amorphous" refers to material that does not have long-range three-dimensional translational order. Partially crystalline materials and crystalline mesophases with e.g. one-or two-dimensional translational order (liquid crystals), or orientational disorder (orientationally disordered crystals), or with conformational disorder (conformationally disordered crystals) are included as well. The drug may be present in the pharmaceutical compositions in an amount ranging from about 1 to about 95 wt%, and most preferably from about 10 to about 80 wt%.

The invention is suitable for compositions containing a drug having virtually any aqueous solubility at physiologically relevant pHs (*i.e.*, pH 1-8), from less than about 0.01 mg/mL, up to about 40 mg/mL and greater.

Exemplary drugs that may be used with the current invention include, without limitation, inorganic and organic compounds that act on the peripheral nerves, adrenergic receptors, cholinergic receptors, nervous system, skeletal muscles, cardiovascular smooth muscles, blood circulatory system, synaptic sites, neuroeffector junctional sites, endocrine and hormone systems, immunological system, reproductive system, autocoid systems, alimentary and excretory systems, inhibitors of autacoids and histamine systems. Preferred classes of drugs include, but are not limited to, Analgesics, antacids, anti-Alzheimer's disease agents, antianginals, antianxiety agents, antiarrhythmics, antiatherosclerotic agents, antibacterials, antibiotics, anticlotting agents, anticonvulsants, antidepressants, antidiarrheals, antiepileptics, antifungals, antihistamines, antihypertensives, anti-impotence agents, anti-inflammatories, antineoplastics, antiobesity agents, anti-Parkinsonism agents, antipsychotic agents, antitussives, antiviral agents, autoimmune disorder agents, beta blockers, blood glucose-lowering agents, cardiac agents, cholesterol-reducing agents, cholesteryl ester transfer protein (CETP) inhibitors, cognitive enhancers, contraceptives, cough suppressants, cytotoxics, decongestants, diuretics, drugs for genito-urinary disorders, drugs for use in rheumatic disorders, glycogen phosphorylase inhibitors, hypnotics, lipid lowering drugs, minerals and vitamins, and sex hormones. Veterinary drugs may also be suitable for use with the present invention.

Each named drug should be understood to include the neutral form of the drug, pharmaceutically acceptable forms of the drug. By "pharmaceutically acceptable forms" is meant any pharmaceutically acceptable derivative or variation, including stereoisomers, stereoisomer mixtures, enantiomers, solvates, hydrates, isomorphs, polymorphs, pseudomorphs, neutral forms, salt forms and prodrugs. Specific examples of antihypertensives include prazosin, nifedipine, amlodipine besylate, trimazosin and doxazosin; specific examples of a blood glucose-lowering agent are glipizide and chlorpropamide; a specific example of an anti-impotence agent is sildenafil and sildenafil citrate; specific examples of antineoplastics include chlorambucil, lomustine and echinomycin; a specific example of an imidazole-type antineoplastic is tubulazole; a specific example of an anti-hypercholesterolemic is atorvastatin calcium; specific examples of anxiolytics include hydroxyzine hydrochloride and doxepin hydrochloride; specific examples of anti-inflammatory agents include betamethasone, prednisolone, aspirin, piroxicam, valdecoxib, carprofen, celecoxib, flurbiprofen and (+)-N-{4-[3-(4-fluorophenoxy)phenoxy]-2-cyclopenten-1-yl}-N-hyroxyurea; a specific example of a barbiturate is phenobarbital; specific examples of antivirals include acyclovir, nelfinavir, and virazole; specific examples of vitamins/nutritional agents include retinol and vitamin E; specific examples of beta blockers include timolol and nadolol; a specific example of an emetic is apomorphine; specific examples of a diuretic include chlorthalidone and spironolactone; a specific example of an anticoagulant is dicumarol; specific examples of cardiotonics include digoxin and digitoxin; specific examples of androgens include 17-methyltestosterone and testosterone; a specific example of a mineral corticoid is desoxycorticosterone; a specific example of a steroidal hypnotic/anesthetic is alfaxalone; specific examples of anabolic agents include fluoxymesterone and methanstenolone; specific examples of antidepression agents include sulpiride, [3,6-dimethyl-2-(2,4,6-trimethyl-phenoxy)-pyridin-4-yl]-(1-ethylpropyl)-amine, 3,5-dimethyl-4-(3'-pentoxy)-2-(2',4',6'-trimethylphenoxy)pyridine, pyroxidine, fluoxetine, paroxetine, venlafaxine and sertraline; specific examples of antibiotics include carbenicillin indanylsodium, bacampicillin hydrochloride, troleandomycin, doxycyline hyclate, ampicillin and penicillin G; specific examples of anti-infectives include benzalkonium chloride and chlorhexidine; specific examples of coronary vasodilators include nitroglycerin and mioflazine; a specific example of a hypnotic is etomidate; specific examples of carbonic anhydrase inhibitors include acetazolamide and chlorzolamide; specific examples of antifungals include econazole, terconazole, fluconazole, voriconazole, and griseofulvin; a specific example of an antiprotozoal is metronidazole; specific examples of anthelmintic agents include thiabendazole and oxfendazole and morantel; specific examples of antihistamines include astemizole, levocabastine, cetirizine, decarboethoxyloratadine, and cinnarizine; specific examples of antipsychotics include ziprasidone, olanzepine, thiothixene hydrochloride, fluspirilene, risperidone and penfluridole; specific examples of gastrointestinal agents include loperamide and cisapride; specific examples of serotonin antagonists include ketanserin and mianserin; a specific example of a serotonin receptor agonists is eletriptan; a specific example of an anesthetic is lidocaine; a specific example of a hypoglycemic agent is acetohexamide; a specific example of an anti-emetic is dimenhydrinate; a specific example of an antibacterial is cotrimoxazole; a specific example of a dopaminergic agent is L-DOPA; specific examples of anti-Alzheimer's Disease agents are THA and donepezil; a specific example of an anti-ulcer agent/H2 antagonist is famotidine; specific examples of sedative/hypnotic agents include chlordiazepoxide and triazolam; a specific example of a vasodilator is alprostadil; a specific example of a platelet inhibitor is prostacyclin; specific examples of ACE inhibitor/antihypertensive agents include enalaprilic acid, quinapril and lisinopril; specific examples of tetracycline antibiotics include oxytetracycline and minocycline; specific examples of macrolide antibiotics include erythromycin, clarithromycin, and spiramycin; a specific example of an azalide antibiotic is azithromycin; specific examples of glycogen phosphorylase inhibitors include [R-(R*S*)]-5-chloro-N-[2-hydroxy-3-{methoxymethylamino}-3-oxo-1-(phenylmethyl)propyl-1H-indole-2-carboxamide and 5-chloro-1H-indole-2-carboxylic acid [(1S)-benzyl-(2R)-hydroxy-3-((3R,4S)-dihydroxy-pyrrolidin-1-yl-)-3-oxypropyl]amide; specific examples of CETP inhibitors include [2R,4S]-4-[acetyl-(3,5-bis-trifluoromethyl-benzyl)-amino]-2-ethyl-6-trifluoromethyl-3,4-dihydro-2H-quinoline-1-carboxylic acid isopropyl ester (torcetrapib), [2R,4S] 4-[(3,5-bis-trifluoromethyl-benzyl)-methoxycarbonyl-amino]-2-ethyl-6-trifluoromethyl-3,4-dihydro-2H-quinoline-1-carboxylic acid ethyl ester, [2R,4S] 4-[(3,5-bis-trifluoromethyl-benzyl)-methoxycarbonyl-amino]-2-ethyl-6-trifluoromethyl-3,4-dihydro-2H-quinoline-1-carboxylic acid isopropyl ester, (2R)-3-[[3-(4-chloro-3-ethylphenoxy)phenyl][[3-(1,1,2,2 tetrafluoroethoxy)phenyl]methyl]amino]-1,1,1-trifluoro-2-propanol, (2R, 4R, 4aS)-4-[amino-(3,5-bis-(trifluoromethyl-phenyl)-methyl]-2-ethyl-6-(trifluoromethyl)-3,4-dihydroquinoline-1-carboxylic acid isopropyl ester, S-[2-([[1-(2-ethylbutyl)cyclohexyl]carbonyl]amino)phenyl]2-methylpropanethioate, trans-4-[[[2-[[[3,5-bis(trifluoromethyl)phenyl]methyl](2-methyl-2H-tetrazol-5-yl)amino]methyl]-4-(trifluoromethyl)phenyl]ethylamino]methyl]-cyclohexaneacetic acid, trans-4-[[[2-[[[3,5-bis(trifluoromethyl)phenyl]methyl](2-methyl-2H-tetrazol-5-yl)amino]methyl]-5-methyl-4-(trifluoromethyl)phenyl]ethylamino]methyl]-cyclohexaneacetic acid; the drugs disclosed in commonly owned U.S. Patent Application Serial Nos. 09/918,127 and 10/066,091, the disclosures of which are incorporated herein by reference; and the drugs disclosed in the following patents and published applications: DE 19741400 A1; DE 19741399 A1; WO 9914215 A1; WO 9914174; DE 19709125 A1; DE 19704244 A1; DE 19704243 A1; EP 818448 A1; WO 9804528 A2; DE 19627431 A1; DE 19627430 A1; DE 19627419 A1; EP 796846 A1; DE 19832159; DE 818197; DE 19741051; WO 9941237 A1; WO 9914204 A1; WO 9835937 A1; JP 11049743; WO 200018721; WO 200018723; WO 200018724; WO 200017164; WO 200017165; WO 200017166; EP 992496; and EP 987251, the disclosures of all of which are incorporated by reference.

## Pharmaceutical Compositions

In one aspect, the invention provides a pharmaceutical composition comprising a drug, the microporous binder described herein, and one or more optional excipients. Preferably, the pharmaceutical composition is in the form of a lightly compressed tablet comprising the drug, the microporous binder of the invention, and optionally one or more tableting excipients, and is characterized by rapid disintegration and/or dissolution in an aqueous medium, i.e., less than about 5 minutes, preferably less than about 2 minutes; robustness or low friability in the dry or solid state; and good, i.e., non-gritty, "mouth feel."

In one embodiment the drug in the pharmaceutical composition is taste-masked. Taste-masking of the drug may be accomplished by any method that effectively masks the taste of the drug when the pharmaceutical composition is placed into the mouth. Examples of taste-masking methods include (1) the inclusion of sweeteners and/or flavorants in the dosage form; (2) coating drug particles to form drug-containing granules, as set forth in U.S. Patent Nos. 4,871,549 and 5,082,669, the disclosures of which are incorporated herein by reference; (3) the inclusion of a molar excess of cyclodextrin to complex the drug, as set forth in copending U.S. Application Serial No. 60/590,803 filed July 22, 2004 and entitled "Improved Taste-Masking With Cyclodextrin Complexes," the disclosure of which is incorporated herein by reference; and (4) the formation of coated drug-containing multiparticulates that provide a short initial delay in dissolution, followed by a rupturing of the multiparticulates in an aqueous environment, as set forth in copending U.S. Application Serial No. 60/561,595, filed April 12, 2004 and entitled "Taste-Masked Drugs in Rupturing Multiparticulates," the disclosure of which is incorporated herein by reference.

Solid dosage forms made with the microporous binders of the present invention may optionally include conventional excipients well known in the pharmaceutical arts. See, for example, Remington: The Science and Practice of Pharmacy (20th Ed. 2000). Generally, excipients such as fillers, disintegrating agents, pigments, lubricants, glidants, flavorants, and so forth may be used for customary purposes. The addition of pH modifiers such as acids, bases, or buffers may also be beneficial, modifying the rate of dissolution of the drug or helping to improve the chemical stability of the compositions.

In one embodiment the dosage form includes one or more taste-masking agents. Examples of taste-masking agents include sweeteners such as aspartame, compressible sugars, dextrates, lactose, mannitol, maltose, sodium saccharin, sorbitol, and xylitol, and flavors such as banana, grape, vanilla, cherry, eucalyptus oil, menthol, orange, peppermint oil, raspberry, strawberry, and watermelon.

Examples of dosage form excipients, fillers, or diluents include lactose, mannitol, xylitol, dextrose, sucrose, sorbitol, compressible sugars, microcrystalline cellulose, powdered cellulose, starch, pregelatinized starch, dextrates, dextran, dextrin, dextrose, maltodextrin, calcium carbonate, dibasic calcium phosphate, tribasic calcium phosphate, calcium sulfate, magnesium carbonate, magnesium oxide, poloxamers such as polyethylene oxide, and hydroxypropyl methyl cellulose.

Examples of surface-active agents include sodium lauryl sulfate and polysorbate 80.

Examples of disintegrants include sodium starch glycolate, sodium carboxymethyl cellulose, calcium carboxymethyl cellulose, croscarmellose sodium, crospovidone (polyvinylpyrrolidone), methylcellulose, microcrystalline cellulose, powdered cellulose, starch, pregelatinized starch, and sodium alginate.

Examples of lubricants include calcium stearate, glyceryl monostearate, glyceryl palmitostearate, hydrogenated vegetable oil, light mineral oil, magnesium stearate, mineral oil, polyethylene glycol, sodium benzoate, sodium lauryl sulfate, sodium stearyl fumarate, stearic acid, talc, and zinc stearate.

Examples of glidants include silicon dioxide, talc and cornstarch.

In one embodiment, a drug and a microporous binder of the present invention are blended together with optional excipients and then compressed to form the dosage form, such as tablets, caplets, or pills. Virtually any process can be used to blend the materials. For example, the compositions can be blended in rotating shell mixers, fixed-shell mixers, planetary paddle mixers, and twin-shell mixers, all known in the art.

The compressed dosage forms may be formed using any of a wide variety of presses used in the fabrication of pharmaceutical dosage forms. Examples include single-punch presses, rotary tablet presses, and multilayer rotary tablet presses, all known in the art. See Remington: The Science and Practice of Pharmacy (20th Ed. 2000). The compressed dosage form may be of any shape, including round, oval, oblong, cylindrical, or triangular. The upper and lower surfaces of the compressed dosage form may be flat, round, concave, or convex.

When formed by compression, the dosage form preferably has a "hardness" of at least about 3 kiloPonds (kP), more preferably at least about 5 kP, and even more preferably at least about 7 kP. Here, "hardness" is the force required to fracture a tablet formed from the materials. The hardness of a tablet may be measured using a Schleuniger Tablet Hardness Tester, model 6D.

The dosage form also preferably has a low friability. Friability is a well-known measure of a dosage form's resistance to surface abrasion that measures weight loss in percentage after subjecting the dosage form to a standardized agitation procedure. Friability values of from 0.8 to 1.0% are regarded as constituting the upper limit of acceptability. Thus, it is preferred that the dosage forms have a friability of less then about 1.0%, more preferably less than about 0.8%.

In one embodiment, the microporous binders of the present invention are used to form compressed dosage forms that rapidly disintegrate when placed in an aqueous use environment. By "rapidly disintegrate" means that the dosage form disintegrates in about 5 minutes or less. More preferably, the dosage form disintegrates in 2 minutes or less, and most preferably in 1 minute or less. The disintegration time is determined according to the USP XXIV disintegration test procedure. In this procedure, a dosage form is placed inside a wire basket, the basket being made from a stainless steel wire cloth with 1.8 to 2.2-mm mesh apertures and a wire diameter of 0.60 to 0.655 mm. The wire basket containing the dosage form is raised and lowered in an immersion fluid at a frequency between 29 and 32 cycles per minute. The immersion fluid (typically water) is held at 37°C. An example of an appropriate apparatus for performing such tests is the Erweka ZT-71 disintegration tester. The disintegration time is the time required to render any residue of the dosage form remaining on the wire basket a soft mass having no palpably firm core, excluding fragments of insoluble coating.

Without further elaboration, it is believed that one of ordinary skill in the art can, using the foregoing description, utilize the present invention to its fullest extent. Therefore, the following exemplary embodiments are to be construed as merely illustrative and not restrictive of the scope of the invention. Those of ordinary skill in the art will understand that variations of the conditions and processes of the following examples can be used.

**Microporous Binder 1**

Microporous binder particles containing 60 wt% HPMCAS (AQOAT-LG, available from Shin Etsu, Tokyo, Japan) and 40 wt% sucrose (Microporous Binder 1) were prepared as follows. First, a spray solution was formed containing 810 g HPMCAS (5.4 wt%), 540 g sucrose (3.6 wt%), 8230 g acetone (55 wt%), and 5400 g (36 wt%) water. The spray solution was pumped using a high-pressure pump to a spray-drier, a Niro type XP Portable Spray-Dryer with a Liquid-Feed Process Vessel Model PSD-1, equipped with a pressure nozzle (Schlick 121 #3.5 60° angle nozzle tip). The PSD-1 was equipped with a 9-inch chamber extension. The spray-drier was also equipped with a diffuser plate having holes therein comprising 1 % of the surface area of the diffuser plate. The pressure nozzle was mounted flush with the diffuser plate during operation. The spray solution was pumped to the spray-drier at about 100 g/min at a pressure of 200 psig. Nitrogen drying gas was directed through the diffuser plate at an inlet temperature of 155°C. The evaporated solvent and drying gas exited the spray-drier at a temperature of 50°C. The resulting solid microporous particles were collected in a cyclone.

The properties of microporous Binder 1 were determined. First, the bulk and tapped specific volume of Microporous Binder 1 were determined using the following procedure. A sample of Microporous Binder 1 was poured into a 1 00-mL graduated cylinder, the tare weight of which had been measured, and the volume and weight of the sample recorded. The volume divided by the weight yielded the bulk specific volume of 7.9 mUg, as shown in Table 1. Next, the cylinder containing Microporous Binder 1 was tapped 1000 times using a VanKel tap density instrument, Model 50-1200. The tapped volume divided by the same weight of Microporous Binder 1 yielded a tapped specific volume of 4.7 mUg.

The volume-weighted mean diameter of the Microporous Binder 1 particles was measured by recording data from laser light scattering using a Malvem Mastersizer 2000, then performing a calculation based on the data. A dry powder feed method was used, and samples were taken at a rate of 3 measurements per aliquot with a delay time of 7 seconds. The dispersive air pressure was 2 barg, and the vibration feed rate was 75% of maximum. Volume-weighted mean diameter was calculated from the light scattering data assuming a gaussian size distribution, with approximately 85% of the particle volume being within about 30% of the reported size. The results are given in Table 1.

The porosity of Microporous Binder 1 was determined by mercury porosimetry as follows. Approximately 100 mg of the binder was added to the bulb of a calibrated 3 cc powder penetrometer of an AutoPore IV Mercury Porosimeter having a threaded closure. The sample was surrounded with mercury, pressure was applied, and the change in mercury volume as a function of pressure was measured. The intrusion volume of mercury as a function of pressure was converted to log differential intrusion volume and plotted against pore size, and the relative contribution of void volume as a function of pore size was quantified. From this test, the porosity of the sample was determined to be 82%. The properties of Microporous Binder 1 are summarized in Table 1.

Table 1

| Properties of Microporous Binder 1 | |
|---|---|
| Bulk Specific Volume (mUg) | 7.9 |
| Tapped Specific Volume (mL/g) | 4.7 |
| Mean Particle Diameter ($\mu$m) | 41 |
| $D_{10}$, $D_{50}$, $D_{90}$ ($\mu$m) | 12, 37, 76 |
| Span $(D_{90}-D_{10})/D_{50}$ | 1.7 |
| Porosity (%) | 82 |
| *10 vol% of the particles had a diameter that is smaller than $D_{10}$; 50 vol% of the particles had a diameter that is smaller than $D_{50}$, and 90 vol% of the particles had a diameter that is smaller than $D_{90}$. | |

FIG. 1 shows a microtomed cross section of Microporous Binder 1 observed using scanning electron microscopy (SEM). As is apparent, Microporous Binder 1 was highly porous.

For comparison, particles were made by dry-blending 60 wt% of the same grade HPMCAS with 40 wt% sucrose and the porosity of the particles was measured in the same manner and determined to be only 66%.

**Microporous Binder 2**

Microporous binder particles containing 80 wt% HPMCAS-LF and 20 wt% sucrose (Microporous Binder 2) were prepared in the same manner as for Microporous Binder 1 with the following exceptions. The spray solution contained 6.8 wt% HPMCAS, 1.95 wt% sucrose, 52.2 wt% acetone, and 39 wt% water. The spray solution was pumped to the PSD-1 spray- drier at from about 140 to about 160 g/min at a pressure of 150 to 180 psig. The drying gas was directed through the diffuser plate at a flow rate of 1800 g/min and an inlet temperature of 210°C. The evaporated solvent and drying gas exited the spray drier at a temperature of 56°C. The porosity of the so-formed particles was measured as described for Microporous Binder 1 and determined to be 75%.

**Compaction Properties of Microporous Binders 1 and 2**

Approximately 250 mg samples of Microporous Binders 1 and 2 (MB 1 and MB 2) were weighed and separately placed in 50% RH and 0% RH chambers to equilibrate their relative dryness overnight. Following equilibration, compacts of MB 1 and MB 2 were formed in triplicate using a Carver press with 15/32" flat-faced, beveled edge tooling, and a dwell time of 5 seconds. Compaction forces of 250, 500, 750, and 1000 psig were applied. Hardness in kiloPonds (kP) was measured using a Schleuniger tablet hardness tester, Model 6D. The void fraction of the compacts was calculated by dividing the measured weight per volume for each compact by the true density of non-compacted powdered binders, and subtracting the fraction from 1 to obtain the void fraction. Thus, the void fraction is the fraction of the compact that does not contain material. Table 2 shows compact hardness and void fraction at varying compaction forces.

Table 2

| Compaction Force (psig) | Hardness (kP) | | | | Void Fraction | | | |
|---|---|---|---|---|---|---|---|---|
| | 0% RH | | 50% RH | | 0% RH | | 50% RH | |
| | MB1 | MB2 | MB1 | MB2 | MB1 | MB2 | MB1 | MB2 |
| 250 | 1.7 | 1.8 | 4.0 | 15.8 | 0.56 | 0.59 | 0.54 | 0.39 |
| 500 | 4.4 | 4.9 | 11.5 | 26.7 | 0.47 | 0.47 | 0.38 | 0.28 |
| 750 | 7.1 | 7.5 | 13.9 | 29.7 | 0.41 | 0.41 | 0.32 | 0.23 |
| 1000 | 10.4 | 9.9 | 17.2 | 32.5 | 0.38 | 0.39 | 0.27 | 0.21 |
| MB1 = 60/40 HPMCAS/sucrose (w/w)  MB2 = 80/20 HPMCAS/sucrose (w/w) | | | | | | | | |

The data in Table 2 show that for both MB 1 and MB 2, hardness increased with compaction force and humidity. However, void fraction decreased with compaction force and humidity.

**Multiparticulates 1** (Not the invention)

Multiparticulates (Multiparticulates 1) comprising 20 wt% cetirizine, 60 wt% glyceryl mono-, di- and tri- behenates (commercially available as COMPRITOL 888® from Gattefosse Corporation of Westwood, New Jersey), 15.0 wt% croscarmellose sodium (commercially available as AcDiSol from FMC of Philadelphia, Pennsylvania), and 5 wt% of poloxamer 407 (commercially available as PLURONIC F127 from BASF of Mount Olive, New Jersey) were prepared using the following procedure. First, 900 g of COMPRITOL® and 75 g of PLURONIC were added to a sealed, jacketed stainless-steel tank (Malto-Mat-Universal MMU 5, Krieger AGG, Switzerland) equipped with counter-rotating mixing paddles and a homogenizer. Heating fluid at 90°C was circulated through the jacket of the tank, and the mixture was melted and stirred. Next, 300 g of cetirizine and 225 g AcDiSol were added to the melt and homogenized for 5 minutes, resulting in a feed suspension of the cetirizine in the molten components.

The feed suspension was then pumped at a rate of 140 g/min using a gear pump (Zenith Pump, Parker Hannifin Corp, Model C-9000, 2.4 cc/rev) to the center of a 4-inch diameter spinning-disk atomizer rotating at 5500 rpm, the surface of which was heated to 90°C. The particles formed by the atomizer were congealed in ambient air and a total of 1410 g of particles were collected.

The particles were then coated with a polymer as follows. A spray solution was prepared by diluting an aqueous ethylcellulose dispersion, Surelease® E-7-7050 (available from Colorcon as an aqueous emulsion containing 25 wt% solids) to 15 wt% solids in water, and adding 10 drops of blue food coloring. The particles were fluidized in a Glatt GPCG-1 fluidized bed coater equipped with a Würster column set at 15 mm. Air fluidizing gas was circulated through the bed at a rate of 35 to 39 ft$^3$/min at an inlet temperature of 55° to 61°C and a bed temperature of 44°C. The spray solution was introduced to the bed through a two-fluid nozzle at a rate of 4.0 to 6.8 g/min using air with an atomization pressure of 2.2 barg. The particles were coated for 162 minutes, resulting in multiparticulates with an average coating weight of 30 wt% (the coating amount was calculated as the weight of coating material applied divided by the final weight of the coated multiparticulate, then multiplied by 100%).

**Cetirizine Release from Multiparticulates 1** (Not the invention)

The rate of release of cetirizine *in vitro* from Multiparticulates 1 was determined using the following procedure. About 70 mg of Multiparticulates 1 were placed into a USP Type 2 dissoette flask equipped with Teflon-coated paddles rotating at 50 rpm. The flask contained 900 mL of simulated mouth buffer (0.05 M $KH_2PO_4$ buffer adjusted to pH 7.3 with KOH) held at 37.0 ± 0.5°C. Samples were taken with 10 μm filters attached to a cannula. A 4-mL sample of the fluid in the flask was drawn and the cannula removed. A 0.45-μm filter was attached to the syringe, 2 mL of sample was returned to the dissolution flask, and 1 mL of sample was filtered into a High Performance Liquid Chromatography (HPLC) vial. The remaining solution in the syringe was drawn from the filter to pull any multiparticulates away from the filter, and returned to the flask. Samples were collected at 1, 2, 3, 5, 10, 20, and 30 minutes following addition of the multiparticulates to the flask. The samples were analyzed using HPLC (Hewlett Packard 1100; Mac Mod Analytical Zorbax Stablebond CN (SB-CN) column, 5 μm particles, 15 cm x 4.6 mm i.d.; 100 mM $KH_2PO_4$, pH 6.5 / MeOH (50/50) with 1 g/L sodium octanesulfonate at 1.0 mL/min; absorbance measured at 214 nm with a diode array spectrophotometer).

The amount of drug released was calculated based on the potency assay of the formulation. To measure the potency of Multiparticulates 1, about 80 mg of the multiparticulates were weighed and added to a 100 mL volumetric flask. Next, 10 mL acetonitrile was added, and the solution was sonicated for 10 minutes. The flask was filled to volume with the HPLC mobile phase noted above, and sonicated for an additional 10 minutes. The solution was then filtered and analyzed to determine the total amount of drug in the formulation. The potency assay of the formulation was used to calculate the amount of drug added for each dissolution test. The amount of drug in each sample was divided by the total amount of drug added for the test, and the results are reported as percent of assay. The results of these dissolution tests are given in Table 3.

Table 3

| Time (min) | Cetirizine Released from Multiparticulates 1 (% assay) |
|---|---|
| 0 | 0 |
| 1 | 2 |
| 2 | 8 |
| 3 | 21 |
| 5 | 52 |
| 10 | 83 |
| 20 | 93 |
| 30 | 93 |

**Dosage Form 1**

MB 2 was used to prepare a dosage form (Dosage Form 1) containing Multiparticulates 1 as follows. Tablets were made containing 30.0 wt% MB 2, 37.0 wt% Multiparticulates 1, 13.8 wt% microcrystalline cellulose (commercially available as Avicel PH200 from FMC of Philadelphia, Pennsylvania), 13.7 wt% lactose, 5.0 wt% AcDiSol, and 0.5 wt% magnesium stearate. To form the tablets, all ingredients except the magnesium stearate were weighed into a container and mixed with a Turbula blender for 20 minutes. Next, the magnesium stearate was added and blended for 4 minutes. The mixture was then weighed into 100 mg samples and formed into tablets using the Manesty F-Press with 3/8" flat-faced, beveled edge tooling. The compression force was set to deliver tablets with a hardness of about 2 kP.

**Cetirizine Release from Dosage Form 1**

The rate of release of cetirizine *in vitro* from Dosage Form 1 was determined as described for Multiparticulates 1. The results are given in Table 4, together with those for Multiparticulates 1 for comparison.

Table 4

| Time (min) | Cetirizine Released from Dosage Form 1 (% assay) | Cetirizine Released from Multiparticulates 1 (% assay) |
|---|---|---|
| 0 | 0 | 0 |
| 1 | 0 | 2 |
| 2 | 18 | 8 |
| 3 | 44 | 21 |
| 5 | 71 | 52 |
| 10 | 87 | 83 |
| 20 | 93 | 93 |
| 30 | 94 | 93 |

The results show that after an initial lag time (desired for tastemasking purposes), the cetirizine was rapidly released from both formulations.

**Dosage Forms 2-3**

Placebo tablets (Dosage Form 2) were made containing 30.0 wt% MB 1, 30.0 wt% Avicel PH101, 29.5 wt% lactose, 10.0 wt% AcDiSol, and 0.5 wt% magnesium stearate. Placebo tablets (Dosage Form 3) were also made containing 30.0 wt% MB 1, 59.5 wt% mannitol, 10.0 wt% AcDiSol, and 0.5 wt% magnesium stearate. Tablet ingredients were blended and formed as for Dosage Form 1. The compression force was set to deliver tablets with a hardness of about 0.5 to 1.0 kP.

*In Vivo* Evaluation of Placebo Dosage Forms 2 and 3

Placebo Dosage Forms 2 and 3 were evaluated in human tests. Six panelists were given each of the Dosage Forms. The protocol consisted of holding the tablet in the mouth until the tablet had disintegrated and then noting the time for disintegration and the mouth feel. For Dosage Form 2 the disintegration time was about 15 seconds, while that for Dosage Form 3 was from about 10 to about 15 seconds.

**Claims**

1.  (amended) 1. A fast-disintegrating dosage form comprising a drug and a microporous binder, said microporous binder being free from said drug and comprising a plurality of particles, each of said particles comprising an aqueous-soluble ionizable cellulosic polymer selected from hydroxypropyl methyl cellulose phthalate, hydroxypropyl methyl cellulose acetate succinate, carboxymethyl ethyl cellulose, cellulose acetate phthalate, cellulose acetate trimellitate, and mixtures thereof and a wicking agent, said wicking agent being selected from the group consisting of polyols, salts, organics, low- molecular-weight polymers, and mixtures thereof, wherein said aqueous-soluble polymer and said wicking agent together comprise at least about 60 wt% of said microporous binder, and wherein said microporous binder has a porosity of at least about 70%.

2.  (original) The dosage form of claim 1 wherein said polyols are selected from the group consisting of sugars, sugar alcohols, and mixtures thereof.

3.  (original) The dosage form of claim 2 wherein said wicking agent is selected from the group consisting of sucrose, glucose, dextrose, lactose, mannitol, sorbitol, trehalose, xylitol, and mixtures thereof.

**4.** (original) The dosage form of claim 1 wherein said polymer is hydroxypropyl methylcellulose acetate succinate and said wicking agent is sucrose.

**5.** (original) The dosage form of claim 1 having a porosity of at least 75%.

**6.** (original) The dosage form of claim 1 wherein said microporous binder has pores with an average diameter of about 10 $\mu$m or less.

**7.** (original) The dosage form of claim 1 wherein said polymer constitutes at least 30 wt% of said microporous binder.

**8.** (original) The dosage form of claim 1 wherein said microporous binder consists essentially of said aqueous-soluble cellulosic polymer and said wicking agent.

**9.** (original) A process for preparing a fast disintegrating microporous binder comprising the steps:

(a) forming a single phase liquid feed solution comprising an aqueous- soluble cellulosic polymer, a wicking agent, a first solvent, and a second solvent, said wicking agent being selected from the group consisting of polyols, salts, organics, low-molecular-weight polymers, and mixtures thereof;
(b) causing said feed solution to undergo phase separation to form two phases; and
(c) removing a substantial portion of said first solvent and said second solvent from said two phases to form said microporous binder; wherein said aqueous-soluble polymer and said wicking agent together comprise at least about 60 wt% of said microporous binder, and wherein said microporous binder has a porosity of at least about 70%.

**10.** (original) The process of claim 9 wherein said polymer is soluble in said first solvent, said wicking agent is soluble in said second solvent, and wherein the volatility of said first solvent is greater than the volatility of said second solvent.

**11.** (original) The process of claim 9 wherein step (b) is conducted by a method selected from (i) altering the ratio of said first solvent to said second solvent; (ii) cooling the feed solution; and (iii) a combination of (i) and (ii).

**12.** (original) The process of claim 9 wherein steps (b) and (c) are conducted by directing said feed solution to a spray-drying apparatus, and atomizing said feed solution into droplets in said spray-drying apparatus.

**13.** (amended) The process of claim 9 wherein said polymer is selected from the group consisting of hydroxypropyl methylcellulose phthalate, hydroxypropyl methylcellulose acetate succinate, carboxymethyl ethylcellulose, cellulose acetate phthalate, cellulose acetate trimellitate, and mixtures thereof; and said wicking agent is selected from the group consisting of sucrose, glucose, dextrose, lactose, mannitol, sorbitol, xylitol, and mixtures thereof.

**14.** (original) The process of claim 9 wherein said polymer is hydroxypropyl methylcellulose acetate succinate and said wicking agent is sucrose.

**Patentansprüche**

**1.** Schnell zerfallende Dosierform mit einem Arzneimittel und einem mikroporösen Bindemittel, wobei das mikroporöse Bindemittel frei von dem Arzneimittel ist und eine Mehrzahl von Teilchen aufweist, und jedes der Teilchen ein wasserlösliches ionisierbares cellulosisches Polymer enthält, das ausgewählt ist aus der Gruppe aus Hydroxypropylmethylcellulosephthalat, Hydroxypropylmethylcelluloseacetatsuccinat, Carboxymethylethylcellulose, Celluloseacetatphthalat, Celluloseacetattrimellithat und Mischungen davon sowie eines Dochtmittels, und das Dochtmittel ausgewählt ist aus der Gruppe bestehend aus Polyolen, Salzen, organischen Verbindungen, Polymeren mit niedrigem Molekulargewicht und Mischungen davon, wobei das wasserlösliche Polymer und das Dochtmittel gemeinsam zumindest etwa 60 Gew.-% des mikroporösen Bindemittels enthalten, und wobei das mikroporöse Bindemittel eine Porosität von zumindest etwa 70% aufweist.

**2.** Dosierform nach Anspruch 1, wobei die Polyole ausgewählt sind aus der Gruppe bestehend aus Zuckern, Zuckeralkoholen, und Mischungen davon.

**3.** Dosierform nach Anspruch 2, wobei das Dochtmittel ausgewählt ist aus der Gruppe bestehend aus Sucrose, Glucose, Dextrose, Lactose, Mannitol, Sorbitol, Trehalose, Xylitol und Mischungen davon.

4. Dosierform nach Anspruch 1, wobei das Polymer Hydroxypropylmethylcelluloseaccetatsuccinat ist, und das Dochtmittel Sucrose ist.

5. Dosierform nach Anspruch 1 mit einer Porosität von zumindest 75%.

6. Dosierform nach Anspruch 1, wobei das mikroporöse Bindemittel Poren mit einem durchschnittlichen Durchmesser von etwa 10 μm oder weniger aufweist.

7. Dosierform nach Anspruch 1, wobei das Polymer zumindest 30 Gew.-% des mikroporösen Bindemittels bildet.

8. Dosierform nach Anspruch 1, wobei das mikroporöse Bindemittel im Wesentlichen aus dem wasserlöslichen cellulosischen Polymer und dem Dochtmittel besteht.

9. Verfahren zur Herstellung eines schnell zerfallenden mikroporösen Bindemittels mit den Schritten:

   (a) Bilden einer einzelphasigen flüssigen Zufuhrlösung mit einem wasserlöslichen cellulosischen Polymer, einem ersten Lösungsmittel und einem zweiten Lösungsmittel, und das Dochtmittel ausgewählt ist aus der Gruppe bestehend aus Polyolen, Salzen, organischen Verbindungen, Polymeren mit niedrigem Molekulargewicht und Mischungen davon;
   (b) Bewirken einer Phasentrennung der Zufuhrlösung, um zwei Phasen zu bilden; und
   (c) Entfernen eines wesentlichen Teiles des ersten Lösungsmittels und des zweiten Lösungsmittels aus den zwei Phasen, um das mikroporöse Bindemittel zu bilden; wobei das wasserlösliche Polymer und das Dochtmittel gemeinsam zumindest etwa 60 Gew.-% des mikroporösen Bindemittels enthalten, und wobei das mikroporöse Bindemittel eine Porosität von zumindest etwa 70% aufweist.

10. Verfahren nach Anspruch 9, wobei das Polymer in dem ersten Lösungsmittel löslich ist, und das Dochtmittel in dem zweiten Lösungsmittel löslich ist, und wobei die Volatilität des ersten Lösungsmittels größer ist als die Volatilität des zweiten Lösungsmittels.

11. Verfahren nach Anspruch 9, wobei Schritt (b) durch ein Verfahren durchgeführt wird, das ausgewählt ist aus (i) Abändern des Verhältnisses von dem ersten Lösungsmittel zu dem zweiten Lösungsmittel; (ii) Kühlen der Zufuhrlösung; und (iii) einer Kombination von (i) und (ii).

12. Verfahren nach Anspruch 9, wobei die Schritte (b) und (c) durchgeführt werden durch Leiten der Zufuhrlösung zu einem Sprühtrocknungsgerät, und Zerstäuben der Zufuhrlösung in Tröpfchen in dem Sprühtrocknungsgerät.

13. Verfahren nach Anspruch 9, wobei das Polymer ausgewählt ist aus der Gruppe bestehend aus Hydroxypropylmethlycellulosephthalat, Hydroxypropylmethylcelluloseacetatsuccinat, Carboxymethylethylcellulose, Celluloseacetatphthalat, Celluloseacetattrimellithat und Mischungen davon, und das Dochtmittel ausgewählt ist aus der Gruppe bestehend aus Sucrose, Glucose, Dextrose, Lactose, Mannitol, Sorbitol, Xylitol und Mischungen davon.

14. Verfahren nach Anspruch 9, wobei das Polymer Hydroxypropylmethylcelluloseacetatsuccinat ist, und das Dochtmittel Sucrose ist.

**Revendications**

1. Forme galénique à désintégration rapide comprenant un médicament et un liant microporeux, ledit liant microporeux étant exempt dudit médicament et comprenant une pluralité de particules, chacune desdites particules comprenant un polymère cellulosique ionisable soluble dans l'eau choisi parmi le phtalate d'hydroxypropyl méthyl cellulose, l'acétate succinate d'hydroxypropyl méthyl cellulose, la carboxyméthyl éthyl cellulose, l'acétate phtalate de cellulose, l'acétate trimellitate de cellulose et des mélanges de ceux-ci et un agent de capillarité, ledit agent de capillarité étant choisi dans le groupe constitué de polyols, de sels, de substances organiques, de polymères de faible masse moléculaire et de mélanges de ceux-ci, où ledit polymère soluble dans l'eau et ledit agent de capillarité comprennent ensemble au moins environ 60 % en poids dudit liant microporeux, et où ledit liant microporeux a une porosité d'au moins environ 70 %.

**2.** Forme galénique selon la revendication 1, dans laquelle lesdits polyols sont choisis dans le groupe constitué de sucres, d'alcools glucidiques et de mélanges de ceux-ci.

**3.** Forme galénique selon la revendication 2, dans laquelle ledit agent de capillarité est choisi dans le groupe constitué du saccharose, du glucose, du dextrose, du lactose, du mannitol, du sorbitol, du tréhalose, du xylitol et de mélanges de ceux-ci.

**4.** Forme galénique selon la revendication 1, dans laquelle ledit polymère est l'acétate succinate d'hydroxypropyl méthyl cellulose et ledit agent de capillarité est le saccharose.

**5.** Forme galénique selon la revendication 1, ayant une porosité d'au moins 75 %.

**6.** Forme galénique selon la revendication 1, dans laquelle ledit mélange microporeux comporte des pores ayant un diamètre moyen d'environ 10 $\mu$m ou moins.

**7.** Forme galénique selon la revendication 1, dans laquelle ledit polymère constitue au moins 30 % en poids dudit liant microporeux.

**8.** Forme galénique selon la revendication 1, dans laquelle ledit liant microporeux est essentiellement constitué dudit polymère cellulosique soluble dans l'eau et dudit agent de capillarité.

**9.** Procédé de préparation d'un liant microporeux à désintégration rapide comprenant les étapes consistant à :

(a) former une solution d'alimentation liquide à phase unique comprenant un polymère cellulosique soluble dans l'eau, un agent de capillarité, un premier solvant et un second solvant, ledit agent de capillarité étant choisi dans le groupe constitué de polyols, de sels, de substances organiques, de polymères de faible masse moléculaire et de mélanges de ceux-ci ;
(b) amener ladite solution d'alimentation à subir une séparation de phase pour former deux phases ; et
(c) éliminer une partie substantielle dudit premier solvant et dudit second solvant desdites deux phases pour former ledit liant microporeux ; où ledit polymère soluble dans l'eau et ledit agent de capillarité comprennent ensemble au moins environ 60 % en poids dudit liant microporeux, et où ledit liant microporeux a une porosité d'au moins environ 70 %.

**10.** Procédé selon la revendication 9 , dans lequel ledit polymère est soluble dans ledit premier solvant, ledit agent de capillarité est soluble dans ledit second solvant, et dans lequel la volatilité dudit premier solvant est supérieure à la volatilité dudit second solvant.

**11.** Procédé selon la revendication 9 , dans lequel l'étape (b) est exécutée par un procédé choisi parmi (i) la modification du rapport dudit premier solvant sur ledit second solvant ; (ii) le refroidissement de la solution d'alimentation ; et (iii) une combinaison de (i) et (ii).

**12.** Procédé selon la revendication 9 , dans lequel les étapes (b) et (c) sont exécutées en dirigeant ladite solution d'alimentation vers un appareil de séchage par pulvérisation, et en atomisant ladite solution d'alimentation en des gouttelettes dans ledit appareil de séchage par pulvérisation.

**13.** Procédé selon la revendication 9 , dans lequel ledit polymère est choisi dans le groupe constitué du phtalate d'hydroxypropyl méthyl cellulose, de l'acétate succinate d'hydroxypropyl méthyl cellulose, de la carboxyméthyl éthyl cellulose, de l'acétate phtalate de cellulose, de l'acétate trimellitate de cellulose et de mélanges de ceux-ci ; et ledit agent de capillarité est choisi dans le groupe constitué du saccharose, du glucose, du dextrose, du lactose, du mannitol, du sorbitol, du xylitol et de mélanges de ceux-ci.

**14.** Procédé selon la revendication 9 ,-dans lequel ledit polymère est l'acétate succinate d'hydroxypropyl méthyl cellulose et ledit agent de capillarité est le saccharose.

# FIG. 1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 1008353 A
- WO 9617579 A
- WO 9852541 A
- US 6177104 B
- WO 9747304 A
- WO 0110418 A1
- WO 2004100857 A
- EP 1369109 A
- US 351568 A
- US 6763607 B
- US 918127 A
- US 10066091 A
- DE 19741400 A1
- DE 19741399 A1
- WO 9914215 A1
- WO 9914174 A
- DE 19709125 A1
- DE 19704244 A1
- DE 19704243 A1
- EP 818448 A1
- WO 9804528 A2
- DE 19627431 A1
- DE 19627430 A1
- DE 19627419 A1
- EP 796846 A1
- DE 19832159
- DE 818197
- DE 19741051
- WO 9941237 A1
- WO 9914204 A1
- WO 9835937 A1
- JP 11049743 B
- WO 200018721 A
- WO 200018723 A
- WO 200018724 A
- WO 200017164 A
- WO 200017165 A
- WO 200017166 A
- EP 992496 A
- EP 987251 A
- US 4871549 A
- US 5082669 A
- US 59080304 P
- US 56159504 P

### Non-patent literature cited in the description

- Remington: The Science and Practice of Pharmacy. 2000
- Perry's Chemical Engineers' Handbook. 1984, 20-5420-57
- **MARSHALL.** Atomization and Spray-Drying. *Chem. Eng. Prog. Monogr.,* 1954, vol. 50
- **MASTERS.** Spray Drying Handbook. 1985
- **LEFEBVRE.** *Atomization and Sprays,* 1989